(19) Europäisches Patentamt
European Patent Office
Office européen des brevets

(11) Numéro de publication: **0 045 251**
**B1**

(12) # FASCICULE DE BREVET EUROPEEN

(45) Date de publication du fascicule du brevet: **30.11.83**

(21) Numéro de dépôt: **81401178.9**

(22) Date de dépôt: **23.07.81**

(51) Int. Cl.³: **C 07 D 513/04,** C 07 D 513/10, A 61 K 31/505 // C07C53/50, (C07D513/04, 277/00, 239/00),(C07D513/10, 277/00, 239/00)

(54) **Nouveaux dérivés de la thiazolo (3,2-a) pyrimidine, leur préparation et les médicaments qui les contiennent.**

(30) Priorité: **24.07.80 FR 8016319**
**15.05.81 FR 8109724**

(43) Date de publication de la demande: **03.02.82 Bulletin 82/5**

(45) Mention de la délivrance du brevet: **30.11.83 Bulletin 83/48**

(84) Etats contractants désignés: **AT BE CH DE FR GB IT LI LU NL SE**

(56) Documents cités: **BE - A - 778 911**

**Le dossier contient des informations techniques présentées postérieurement au dépôt de la demande et ne figurant pas dans le présent fascicule.**

(73) Titulaire: **RHONE-POULENC SANTE, Les Miroirs 18 Avenue d'Alsace, F-92400 Courbevoie Cedex (FR)**

(72) Inventeur: **Debarre, François, 59, rue Adolphe Pajeaud, F-92160 Antony (FR)**
Inventeur: **Fabre, Jean-Louis, 9, rue Fagon, F-75013 Paris (FR)**
Inventeur: **Farge, Daniel, 30, rue des Pins Sylvestres, F-94320 Thiais (FR)**
Inventeur: **James, Claude, 31 bis, avenue Gambetta, F-75020 Paris (FR)**

(74) Mandataire: **Gaumont, Robert et al, RHONE-POULENC RECHERCHES Service Brevets Pharma 25, Quai Paul Doumer, F-92408 Courbevoie Cedex (FR)**

EP 0 045 251 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

BUNDESDRUCKEREI BERLIN

Nouveaux dérivés de la thiazolo (3,2-a) pyrimidine, leur préparation et les médicaments qui les contiennent

La présente invention concerne de nouveaux dérivés de la tétrahydro-2,3,6,7 thiazolo [3,2-a] pyrimidinone-5 de formule générale:

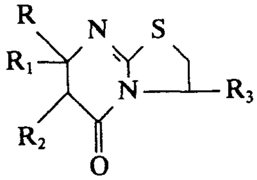

(I)

leur préparation et les médicaments qui les contiennent.

Dans la formule générale (I):

– soit R représente un radical phényle, alcoyle contenant 1 à 4 atomes de carbone non substitué ou substitué par 1 à 3 atomes d'halogène, alcényle contenant 2 à 4 atomes de carbone ou cycloalcoyle contenant 3 à 6 atomes de carbone et $R_1$ représente un atome d'hydrogène, ou bien R et $R_1$, identiques ou différents, représentent chacun un radical phényle ou alcoyle contenant 1 à 4 atomes de carbone non substitué, ou forment ensemble un radical alcoylène contenant 4 ou 5 atomes de carbone et $R_2$ et $R_3$, identiques ou différents, représsentent un atome d'hydrogène ou un radical alcoyle contenent 1 à 4 atomes de carbone,

– soit R et $R_1$ représentent chacun un atome d'hydrogène et $R_2$ et $R_3$ représentent l'un un atome d'hydrogène et l'autre un radical alcoyle contenant 1 à 4 atomes de carbone ou bien représentent chacun un tel radical alcoyle.

Il est entendu que les radicaux alcoyles cités ci-dessus ou qui seront cités dans la suite sont en chaîne droite ou ramifiée.

Selon l'invention les produits de formule générale (I) peuvent être préparés par action d'un halogénure de formule générale:

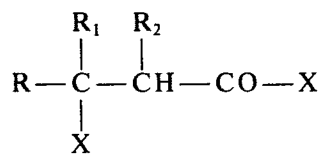

(II)

sur une amino-2 thiazoline-2 (en équilibre avec sa forme imine) de formule générale:

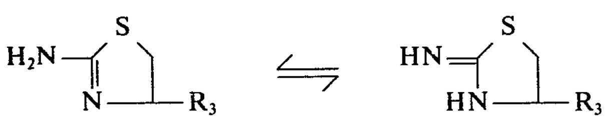

(III)

dans lesquelles R, $R_1$, $R_2$ et $R_3$ sont définis comme ci-dessus et X représente un atome d'halogène tel que le chlore ou le brome.

On opère généralement dans un solvant organique aromatique (par exemple benzène, toluène, xylènes), un solvant chloré (par exemple chloroforme, tétrachlorure de carbone, dichloro-1,2 éthane), un éther (par exemple dioxanne, tétrahydrofuranne) ou le diméthylformamide, à une température comprise entre environ 20°C et la température de reflux du mélange réactionnel en présence d'un accepteur d'acide tel qu'une base minérale (par exemple bicarbonate alcalin) ou organique (par exemple triéthylamine).

Les halogénures de formule générale (II) peuvent être préparés, par exemple, par action d'un agent d'halogénation tel que le chlorure de thionyle sur le $\beta$-hydroxy acide correspondant.

Les amino-2 thiazoline-2 de formule générale (III) peuvent être préparées selon la méthode décrite par S. GABRIEL, Ber. 22, 1141 (1889) ou par S. GABRIEL et H. OHLE, Ber. 50, 804 Selon l'invention, les produits de formule générale (I), à l'exception de ceux pour lesquels R et $R_1$ forment ensemble un radical alcoylène, peuvent encore être préparés selon l'un des procédés suivants:

– par action d'un halogénure d'acide de formule générale:

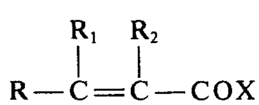

(IV)

sur une amino-2 thiazoline-2 de formule générale (III) dans lesquelles R, $R_1$, $R_2$ et $R_3$ ont les définitions correspondantes et X représente un atome d'halogène tel que le chlore ou le brome.

On opère généralement dans un solvant organique aromatique (par exemple benzène, toluène, xylènes), un solvant chloré (par exemple chloroforme, tétrachlorure de carbone, dichloro-1,2 éthane), un éther (par exemple dioxanne, tétrahydrofuranne) ou le diméthylformamide, à une température comprise entre environ 20° C et la température de reflux du mélange réactionnel en présence d'un accepteur d'acide tel qu'une base minérale (bicarbonate alcalin) ou organique (triéthylamine).

Les halogénures de formule générale (IV) peuvent être préparés à partir des acides correspondants par toute méthode connue en soi n'affectant pas le reste de la molécule:

– par action d'un ester de formule générale:

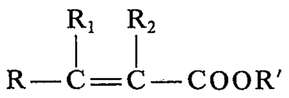

(V)

sur une amino-2 thiazoline-2 de formule (III) dans lesquelles R, $R_1$, $R_2$ et $R_3$ ont les définitions correspondantes et R' représente un radical alcoyle contenant 1 à 4 atomes de carbone.

On opère généralement sans solvant ou bien dans un solvant organique aromatique tel que benzène, toluène ou xylènes à une température comprise entre environ 50° C et la température de reflux du mélange réactionnel, en présence d'un antioxydant tel que l'hydroquinone.

Les esters de formule générale (V) peuvent être obtenus à partir des acides correspondants par toute méthode connue en soi pour obtenir un ester acrylique.

Selon l'invention, les produits de formule générale (I) dans lesquels $R_3$ représente un atome d'hydrogène peuvent aussi être préparés par action du dibromo-1,2 éthane sur un dihydro-4,5 thio-2 uracile de formule générale:

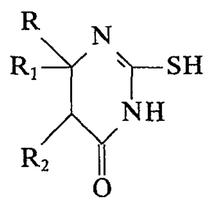

(VI)

dans laquelle R est défini comme précédemment, dans un solvent organique anhydre tel que le diméthylformamide à une température comprise entre environ −10 et 25° C maximum en présence d'un hydrure alcalin tel que l'hydrure de sodium.

Les dihydro-4,5 thio-2 uraciles de formule générale (VI) peuvent être préparés selon la méthode décrite par L. S. SANDAKHCHEIEV et V. P. MAMAEV, Izvest. Sibir. Otdel. Akad. Nauk. S.S.S.R. (1961) N° 7, pages 72–76; C. A. 56 24 482.

Les nouveaux produits de formule générale (I) peuvent être purifiés par des méthodes habituelles telles que la cristallisation et la chromatographie.

On connait par le brevet belge 778 911 des dihydro-2,3 thiazolo [3,2-a] pyrimidinones-5; ces produits sont décrits comme ayant une activité analgésique.

Les nouveaux produits de formule générale (I) présentent d'intéressantes propriétés pharmacologiques les rendant utiles pour le traitement de fond de la maladie rhumatismale et également comme anti-allergiques. Ils possèdent en outre des propriétés analgésiques.

Ils sont en particulier actifs à des doses comprises entre 10 et 100 mg/kg par voie orale chez le rat dans l'oedème de la patte provoqué par une réaction d'ARTHUS inversée [oedème provoqué par injection sous-plantaire de 50 µg de sérum de lapin antiovalbumine suivie immédiatement par l'injection intraveineuse d'ovalbumine selon la technique de D.K. GEMMELL et Coll., Agents et Actions, 9, 107 (1979)].

Ils sont également actifs chez le rat à des doses généralement comprises entre 5 et 100 mg/kg par jour pendant 7 jours par voie orale vis-à-vis de l'oedème de la patte provoqué par injection sous-plantaire de Bordetella pertussis (Hemophilus pertussis) chez le rat sensibilisé à cet antigène selon la technique de P.A. DIEPPE et Coll., Agents et Actions, 6, 618 (1976).

Certains d'entre eux se sont montrés actifs à des doses comprises entre 20 et 80 mg/kg par jour pendant 18 jours par voie orale dans la technique de la polyarthrite expérimentale du rat provoquée par injection sous-plantaire, dans une patte postérieure, d'adjuvant complet de FREUND selon F. DELBARRE et Coll., C.R. Soc. Biol. 162, 58 (1968).

L'activité analgésique se manifeste chez la souris à des doeses comprises entre 1 et 100 mg/kg par voie orale dans la technique de E. SIEGMUND et Coll., Proc. Soc. Exp. Biol. Med. 95, 729 (1957).

De plus, les produits selon l'invention présentent une faible toxicité. Chez la souris, la toxicité aiguë (exprimée par sa $DL_{50}$) est généralement comprise entre 300 et 900 mg/kg ou supérieure à 900 mg/kg par voie orale.

Sont particulièrement intérressants les produits de formule générale (I) dans laquelle R représente un radical alcoyle non substitué, phényle ou cycloalcoyle et $R_1$ représente un atome d'hydrogène, ou

bien R et $R_1$ forment ensemble un radical alcoylène et $R_2$ et $R_3$ représentent un atome d'hydrogène ou un radical méthyle.

Sont plus particulièrement intéressants les produits de formule générale (I) dans laquelle R représente un radical alcoyle contenant 1 ou 2 atomes de carbone, phényle ou cyclohexyle et $R_1$ représente un atome d'hydrogène, ou bien R et $R_1$ forment ensemble le radical pentaméthylène, $R_2$ représente un atome d'hydrogène et $R_3$ représente un atome d'hydrogène ou le radical méthyle.

Sont d'un intérêt tout particulier:

- la méthyl-7 oxo-5 tétrahydro-2,3,6,7 5H-thiazolo [3,2-a] pyrimidine
- l'éthyl-7 oxo-5 tétrahydro-2,3,6,7 5H-thiazolo [3,2-a] pyrimidine
- la cyclohexyl-7 oxo-5 tétrahydro-2,3,6,7 5H-thiazolo [3,2-a] pyrimidine
- l'oxo-5 tétrahydro-2,3,6,7 spiro [[5H-thiazolo [3,2-a] pyrimidine-7,1'-cyclohexane]]
- la diméthyl-3,7 oxo-5-tétrahydro-2,3,6,7 5H-thiazolo [3,2-a] pyrimidine
- la phényl-7 oxo-5 tétrahydro-2,3,6,7 5H-thiazolo [3,2-a] pyrimidine.

Les exemples suivants, donnés à titre non limitatif, montrent comment l'invention peut être mise en pratique.

## Exemple 1

Une solution de 3,33 g de chlorure de cinnamoyle dans 1,7 cm³ de chloroforme est ajoutée goutte à goutte en 3 minutes à une température voisine de 20°C à une solution de 2,04 g d'amino-2 thiazoline-2 et de 2,8 cm³ de triéthylamine dans 7,2 cm³ de chloroforme. Le mélange réactionnel, dont la température est alors de 30°C, est ensuite chauffé au reflux pendant 1 heure 40 minutes, puis refroidi à 4°C à l'aide d'un bain de glace. Les cristaux apparus sont séparés par filtration, lavés 2 fois par 5 cm³ au total de chloroforme et éliminés. Les filtrats organiques sont réunis et lavés 2 fois par 5 cm³ au total d'eau distillée, séchés sur du sulfate de sodium anhydre, additionnés de 0,2 g de noir décolorant, filtrés et concentrés jusqu'à un volume de 9 cm³. Le concentrat est chromatographié sur une colonne de 1,5 cm de diamètre contenant 28 g d'alumine neutre (0,12 – 0,15 mm). On élue par un mélange chloroforme/cyclohexane (40 – 60 en volumes) en recueillant des fractions de 50 cm³. Les 4 premières fractions sont réunies et concentrées à sec sous pression réduite (20 mm de mercure; 2,7 kPa) à 40°C. On obtient ainsi 1,1 g de produit brut que l'on dissout dans 1,35 cm³ d'éthanol bouillant. Après refroidissement à 4°C pendant 1 heure, les cristaux apparus sont séparés par filtration, lavés 2 fois par 0,4 cm³ au total d'éthanol refroidi à environ 5°C et séchés sous pression réduite (20 mm de mercure; 2,7 kPa) à une température voisine de 20°C en présence de potasse en pastilles. On obtient ainsi 0,6 g d'oxo-5 phényl-7 tétrahydro-2,3,6,7 5H-thiazolo [3,2-a] pyrimidine sous forme d'un solide blanc fondant à 115°C.

## Exemple 2

Un mélange de 299,5 g de cinnamate d'éthyle, 173,4 g d'amino-2 thiazoline-2 et de 2 g d'hydroquinone est chauffé chauffé à 110°C pendant 24 heures. Après refroidissement à une température voisine de 20°C, on ajoute 800 cm³ de chlorure de méthylène et extrait 5 fois par 2500 cm³ au total d'une solution aqueuse d'acide chlorhydride 2N. Les solutions aqueuses réunies sont amenées à pH 8 par addition d'une solution aqueuse de soude 10N, puis extraites 4 fois par 2000 cm³ au total de chlorure de méthylène. Les extraits organiques sont réunis et séchés sur du sulfate de sodium anhydre, filtrés et concentrés à sec sous pression réduite (20 mm de mercure; 2,7 kPa) à 40°C. Le résidu obtenu (113 g) est chromatographié sur une colonne de 7,5 cm de diamètre contenant 1100 g de silice (0,06 – 0,2 mm). On élue par du chlorure de méthylène en recueillant des fractions de 1000 cm³. Les 2 premières fractions sont éliminées et les 14 fractions suivantes sont réunies et concentrées à sec sous pression réduite (20 mm de mercure; 2,7 kPa) à 40°C. On obtient ainsi 26 g de produit brut que l'on dissout dans 75 cm³ d'éthanol bouillant additionnés de 1 g de noir décolorant. Après filtration à chaud, le filtrat est refroidi à 4°C pendant 12 heures. Les cristaux apparus sont séparés par filtration, lavés 2 fois par 20 cm³ au total d'éthanol refroidi à environ 5°C et séchés sous pression réduite (20 mm de mercure; 2,7 kPa) à une température voisine de 20°C en présence de potasse en pastilles. On obtient ainsi 21,6 g d'oxo-5 phényl-7 tétrahydro-2,3,6,7 5H-thiazolo [3,2-a] pyrimidine sous forme de cristaux blancs fondant à 113°C.

## Exemple 3

51,7 g d'hydrure de sodium en dispersion à 54,5% dans l'huile sont ajoutés en 25 minutes à une solution refroidie à 8°C de 110 g de phényl-4 dihydro-4,5 thio-2 uracile dans 1200 cm³ de

diméthylformamide anhydre en veillant à ce que la température du mélange réactionnel reste inférieure à 15° C. Vingt-cinq minutes après la fin de l'addition, on coule goutte à goutte en 30 minutes 91,7 $cm^3$ de dibromo-1,2 éthane dans le mélange réactionnel en maintenant toujours la température au-dessous de 15°C. On poursuit l'agitation pendant encore 2 heures, puis on laisse revenir la température du mélange à environ 20° C. Le mélange réactionnel est alors amené à pH 5 par addition d'une solution aqueuse d'acide chlorhydrique 1N puis concentré à sec sous pression réduite (1 mm de mercure; 0,13 kPa) à une température à 50° C. Le résidu est repris par 500 $cm^3$ d'eau distillée, amené à pH 9 à l'aide d'une solution aqueuse de soude 10N, et extrait 3 fois par 1400 $cm^3$ au total de chlorure de méthylène. La phase organique ainsi obtenue est lavée par 300 $cm^3$ d'eau distillée, séchée sur du sulfate de sodium anhydre, filtrée et concentrée à sec sous pression réduite (20 mm de mercure; 2,7 kPa) à 40° C. Le résidu obtenu (111 g) est dissous dans 1100 $cm^3$ de chlorure de méthylène, et chromatographié sur une colonne de 6,8 cm de diamètre contenant 1100 g d'alumine basique (0,05 – 0,16 mm). On élue par du chlorure de méthylène en recueillant des fractions de 250 $cm^3$. Les 3 premières fractions sont éliminées; les 3 fraktions suivantes sont réunies et concentrées à sec sous pression réduite (20 mm de mercure; 2,7 kPa) à 40° C. Le résidu obtenu (32,5 g) est dissous dans un mélange bouillant de 70 $cm^3$ d'éthanol et de 70 $cm^3$ d'oxide d'isopropyle. Après refroidissement, les cristaux apparus sont séparés par filtration et séchés sous pression réduite (20 mm de mercure; 2,7 kPa) à une température voisine de 20° C. On obtient ainsi 13,6 g de produit brut, fondant à 110° C, qui sont réunis à 1,4 g de produit préparé de la même dans une autre opération et dissous dans 60 $cm^3$ d'éthanol bouillant. La solution est filtrée à chaud et le filtrat est refroidi à 4° C pendant 1 heure. Les cristaux apparus sont séparés par filtration, lavés par 5 $cm^3$ d'éthanol puis par 5 $cm^3$ d'oxyde d'isopropyle et séchés sous pression réduite (20 mm de mercure; 2,7 kPa) à une température voisine de 20° C en présence de potasse en pastilles. On obtient ainsi 13 g d'oxo-5 phényl-7 tétrahydro-2,3,6,7 5H-thiazolo [3,2-a] pyrimidine sous forme de cristaux blancs fondant à 112° C.

Exemple 4

Une solution de 440 g de chlorure de crotonoyle dans 1200 $cm^3$ de chloroforme est ajoutée goutte à goutte en 47 minutes à une solution de 504 g d'amino-2 thiazoline-2 et de 590 $cm^3$ de triéthylamine dans 1200 $cm^3$ de chloroforme. Le Mélange réactionnel, dont la température est alors de 63° C, est maintenu au reflux pendant 16 heures. Après refroidissement à 20° C environ, les cristaux apparus sont séparés par filtration, lavés 2 fois par 100 $cm^3$ au total de chloroforme et éliminés. Les filtrats organiques sont réunis, concentrés à sec sous pression réduite (20 mm de mercure; 2,7 kPa) à 40° C et ripris par 700 $cm^3$ d'acétate d'éthyle. Les cristaux apparus sont séparés par filtration, lavés par 200 $cm^3$ d'un mélange d'acétate d'éthyle et de méthanol (90 – 10 en volumes) et éliminés. Les filtrats organiques sont réunis et chromatographiés sur une colonne de 9 cm de diamètre contenant 3,5 kg de silice (0,06 – 0,2 mm) en éluant par de l'acétate d'éthyle et en recueillant des fractions de 1000 $cm^3$. Les 5 premières fractions sont éliminées; les 6 fractions suivantes sont réunies et concentrées à sec sous pression réduite (20 mm de mercure; 2,7 kPa) à 40° C. On obtient ainsi 124 g de produit brut fondant à 91° C qui sont divisés en 3 lots égaux pour être chromatographiés séparément, chacun sur une colonne de 6 cm de diamètre contenant 480 g de silice (0,040 – 0,063 mm). On élue par de l'acétate d'éthyle sous une pression de 0,5 bar (51 kPa) en recueillant des fraktions de 200 $cm^3$. Les 8 premières fraktions sont éliminées; les 10 fraktions suivant sont réunies et concentrées à sec sous pression réduite (20 mm de mercure; 2,7 kPa) à 40° C. On obtient ainsi 109 g de produit, sous forme de cristaux blancs fondant à 93° C, que l'on dissout dans 160 $cm^3$ d'acétate d'éthyle bouillant additionnés de 1 g de noir décolorant. Après filtration à chaud, le filtrat est refroidi à 4° C pendant 2 heures; les cristaux apparus sont séparés par filtration, lavés 3 fois par 100 $cm^3$ au total d'oxyde d'isopropyle refroidi à 5° C environ, et séchés sous pression réduite (20 mm de mercure; 2,7 kPa) à une température voisine de 20° C en présence de potasse en pastilles. On obtient ainsi 96,8 g de méthyl-7 oxo-5 tétrahydro-2,3,6,7 5H-thiazolo [3,2-a] pyrimidine sous forme de cristaux blancs fondant à 96° C.

Exemple 5

Une solution de 50 g de chlorure de pentène-2 oyle dans 300 $cm^3$ de chloroforme est ajoutée goutte à goutte en 30 minutes à une solution de 50,6 g d'amino-2 thiazoline-2 et de 59 $cm^3$ de triéthylamine dans 350 $cm^3$ de chloroforme. Le mélange réactionnel, dont la température est alors de 49° C, est chauffé au reflux pendant 16 heures. Après refroidissement à 20° C environ, les cristaux apparus sont séparés par filtration et éliminés. Le filtrat organique est concentré à sec sous pression réduite (20 mm de mercure; 2,7 kPa) à 40° C puis repris par 250 $cm^3$ d'acétone. Les cristaux apparus sont séparés par filtration, lavés daux fois par 60 $cm^3$ au total d'acétone et éliminés. Les filtrats organiques sont concentrés à sec sous pression réduite (20 mm de mercure; 2,7 kPa) à 40° C et chromatographiés sur une colonne de 7 cm de diamètre contenant 820 g de silice (0,06 – 0,2 mm) en éluant par de l'acétate d'éthyle et en recueillant des fractions de 1000 $cm^3$. La première fraction est eliminée; les 4 fractions suivantes sont réunies et

concentrées à sec sous pression réduite (20 mm de mercure; 2,7 kPa) à 40° C. On obtient ainsi 31 g de produit brut sous forme d'huile, qui sont additionnés à 6 g produit préparé de la même manière dans une autre opération pour être chromatographiés sur une colonne de 6 cm de diamètre contenant contenant 480 g de silice (0,040 – 0,063 mm). On élue par un mélange acétate d'éthyle/cyclohexane (70/30 en volumes) sous une pression de 0,5 bar (51 kPa) en recueillant des fractions de 200 $cm^3$. Les 6 premières fractions sont éliminées; les 6 fractions suivantes sont réunies et concentrées à sec sous pression réduite (20 mm de mercure; 2,7 kPa) à 40° C. On obtient ainsi 21,7 g de produit que l'on dissout dans 50 $cm^3$ d'oxyde d'isopropyle bouillant additionnés de 0,2 g de noir décolorant. Après filtration à chaud, le filtrat est refroidi à 4° C pendant 1 heure et demie; les cristaux apparus sont séparés par filtration, lavés 2 fois par 20 $cm^3$ au total d'oxyde d'isopropyle refroidi à 5° C environ, et séchés sous pression réduite (20 mm de mercure; 2,7 kPa) à une température voisine de 20° C en présence de potasse en pastilles. On obtient ainsi 18,6 g d'éthyl-7 oxo-5 tétrahydro-2,3,6,7 5H-thiazolo [3,2-a] pyrimidine sous forme de cristaux blancs fondant à 60° C.

## Exemple 6

Une solution de 35,5 g de chlorure de méthyl-4 pentène-2 oyle dans 200 $cm^3$ de chloroforme est ajoutée goutte à goutte en 20 minutes à une solution de 32,2 g d'amino-2 thiazoline-2 et de 37,7 $cm^3$ de triéthylamine dans 200 $cm^3$ de chloroforme. Le mélange réactionnel, dont la température est alors de 53° C, est chauffé au reflux pendant 16 heures. Après refroidissement à 20° C environ, les cristaux apparus sont séparés par filtration et éliminés. Le filtrat organique est concentré à sec sous pression réduite (20 mm de mercure; 2,7 kPa) à 40° C et le résidu est repris par 200 $cm^3$ d'acétone. Les cristaux apparus sont séparés par filtration, lavés 2 fois par 60 $cm^3$ au total d'acétone et éliminés. Les filtrats organiques sont concentrés à sec sous pression réduite (20 mm de mercure; 2,7 kPa) à 40° C et chromatographiés sur une colonne de 4 cm de diamètre contenant 550 g d'alumine neutre (0,12 – 0,15 mm). On élue par de l'acétate d'éthyle en recueillant des fractions de 500 $cm^3$. La première fraction est éliminée; les 2 fractions suivantes sont réunies et concentrées à sec sous pression réduite (20 mm de mercure; 2,7 kPa) à 40° C. On obtient ainsi 25 g de produit brut sous forme d'huile qui sont chromatographiés à nouveau sur une colonne de 6 cm de diamètre contenant 480 g de silice (0,040 – 0,063 mm). On élue par un mélange acétate d'éthyle/cyclohexane (60/40 en volumes) sous une pression de 0,5 bar (51 kPa) en recueillant des fractions de 250 $cm^3$. Les 7 premières fractions sont éliminées; les 9 fractions suivantes sont réunies et concentrées à sec sous pression réduite (20 mm de mercure; 2,7 kPa) à 40° C. On obtient ainsi 15 g de produit que l'on dissout dans 50 $cm^3$ d'oxyde d'isopropyle bouillant additionnés de 0,2 g de noir décolorant. Après filtration à chaud, le filtrat est refroidi à 4° C pendant 1 heure; les cristaux apparus sont séparés par filtration, lavés 2 fois par 20 $cm^3$ au total d'oxyde d'isopropyle refroidi à environ 5° C et séchés sous pression réduite (20 mm de mercure; 2,7 kPa) à une température voisine de 20° C en présence de potasse en pastilles. On obtient ainsi 11,8 g d'isopropyl-7 oxo-5 tétrahydro-2,3,6,7 5H-thiazolo [3,2-a] pyrimidine sous forme de cristaux blancs fondant à 50,6° C.

## Exemple 7

Une solution de 79,5 g de chlorure d'hex'ene-2 oyle dans 750 $cm^3$ de chloroforme est ajoutée goutte à goutte en 75 minutes à une solution de 61,2 g d'amino-2 thiazoline-2 et de 84,2 $cm^3$ de triéthylamine dans 600 $cm^3$ de chloroforme. Le mélange réactionnel, dont la température est alors de 40° C, est chauffé au reflux pendant 45 minutes, puis après refroidissement à 20° C environ, lavé 4 fois par 1200 $cm^3$ au total d'eau distilée; la phase organique est séchée sur du sulfate de magnésium anhydre, additionnée de 10 g de noir décolorant, filtrée et concentrée à sec sous pression réduite (20 mm de mercure; 2,7 kPa) à 40° C. On obtient ainsi un résidu huileux (110 g) qui est chromatographié sur une colonne de 5,1 cm de diamètre contenant 1,1 kg d'alumine (0,12 – 0,15 mm). On élue par du chlorure de méthylène en recueillant 1000 $cm^3$ d'éluat. Cet éluat est concentré à sec sous pression réduite (20 mm de mercur; 2,7 kPa) à 40° C. On obtient ainsi 25 g de produit que l'on chromatographie à nouveau sur une colonne de 3,2 cm de diamètre contenant 250 g d'alumine (0,12 – 0,15 mm) en présence de cyclohexane. On élue par des mélanges de chloroforme/cyclohexane en recueillant des fractions de 200 $cm^3$. Les 3 premières fractions (solvant d'élution: chloroforme/cyclohexane 20/80 en volumes) sont éliminées; les 4 fractions suivantes (solvant d'élution : chloroforme/cyclohexane 30/70 en volumes) et les 3 dernières fractions (solvant d'élution : chloroforme/cyclohexane 40/60 en volumes) sont réunies et concentrées à sec sous pression réduite (20 mm de mercure; 2,7 kPa) à 40° C. On obtient ainsi un résidu (14,7 g) que l'on reprend par 50 $cm^3$ d'éther éthylique et filtre. Après filtration, la solution est coincentrée à sec sous pression réduite (20 mm de mercure; 2,7 kPa) à 40° C et chromatographiée sur une colonne de 2,4 cm de diamètre contenant 100 g d'alumine (0,12 – 0,15 mm). On élue par fractions de 100 $cm^3$ dans les conditions suivantes:

– Fraction 1: (cyclohexane pur)
– Fraktion 2: (cyclohexane pur)
– Fraktions 3 et 4: (cyclohexane/chloroforme: 90/10 en volumes)
– Fractions 5 et 6: (cyclohexane/chloroforme: 80/20 en volumes)
– Fractions 7 et 8: (cyclohexane/chloroforme: 70/30 en volumes)

La fraction 1 est éliminée; les fractions 2 à 8 sont réunies et concentrées à sec sous pression réduite (20 mm de mercure; 2,7 kPa) à 40° C. On obtient ainsi 4,3 g de produit que l'on dissout dans 20 $cm^3$ d'éther de pétrole (P.E. 40–65° C) bouillant. Après refroidissement à 4° C pendant 16 heures, les cristaux apparus sont séparés par filtration, lavés par 5 $cm^3$ d'éther de pétrole (P.E. 40–65° C) refroidi à environ 5° C et séchés sous pression réduite (20 mm de mercure; 2,7 kPa) à une température voisine de 20° C en présence de potasse en pastilles. On obtient ainsi 2,5 g d'oxo-5 propyl-7 tétrahydro-2,3,6,7 5H-thiazolo [3,2-a] pyrimidine sous forme de craistaux blancs fondant à 47° C.

## Exemple 8

Une solution de 13,1 g de chlorure de t.butyl-3 acryloyle dans 60 cm3 de chloroforme est ajoutée goutte à goutte en 25 Minutes à une solution de 10,25 g d'amino-2 thiazoline-2 et de 12,6 $cm^3$ de triéthylamine dans 60 $cm^3$ de chloroforme. Le mélange réactionnel, dont la température est alors de 51° C, est chauffé au reflux pendans 16 heures. Après refroidissement à 20° C environ, les cristaux apparus sont séparés par filtration et éliminés. Le filtrat organique est concentré à sec sous pression réduite (20 mm de mercure; 2,7 kPa) à 35° C puis repris par 110 $cm^3$ d'acétone. Les cristaux apparus sont séparés par filtration, lavés 2 fois par 50 $cm^3$ au total d'acétone et éliminés. Les filtrats organiques sont réunis et concentrés à sec sous pression réduite (20 mm de mercure; 2,7 kPa) à 30° C. Le résidu est chromatographié sur une colonne de 3,5 cm de diamètre contenant 200 g de silice (0,063–0,2 mm) en éluant par un mélange d'acétate d'éthyle et de cyclohexane (50/50 en volumes) et en recueillant des fractions de 50 $cm^3$. Les 3 premières fractions sont éliminées, les 6 fraktions suivantes sont réunies et concentrées à sec sous pression réduite (20 mm de mercure; 2,7 kPa) à 30° C. On obtient ainsi 4 g de produit brut qui sont additionnés à 2 g de produit préparé de la même manière dans une autre opération et dissous dans 24 $cm^3$ d'oxyde d'isopropyle bouillant additionnés de 0,1 g de noir décolorant. Après filtration à chaud, le filtrat est refroidi à 4° C pendant 2 heures; les cristaux apparus sont séparés par filtration, lavés 2 fois par 10 $cm^3$ au total d'oxyde d'isopropyle refroidi à 5° C environ, et séchés sous pression réduite (20 mm de mercure; 2,7 kPa) à une température voisine de 20° C en présence de potasse en pastilles. On obtient ainsi 4,3 g de t.butyl-7 oxo-5 tétrahydro-2,3,6,7 5H-thiazolo [3,2-a] pyrimidine sous forme de cristaux blancs fondant à 112° C.

## Exemple 9

Une solution de 142,5 g de chlorure de méthyl-5 hexène-2 oyle dans 600 $cm^3$ de chloroforme est ajoutée goutte à goutte en 35 minutes à une solution de 106 g d'amino-2 thiazoline-2 et de 124 $cm^3$ de triéthylamine dans 700 $cm^3$ de chloroforme. Le mélange réactionnel, dont la température est alors de 63° C, est chauffé au reflux pendant 16 heures. Après refoidissement à 20° C environ, les cristaux apparus sont séparés par filtration et éliminés. Le filtrat organique est concentré à sec sous pression réduite (20 mm de mercure; 2,7 kPa) à 45° C puis repris par 1000 $cm^3$ d'acétone. Les cristaux apparus sont séparés par filtration et éliminés. Le filtrat organique est concentré à sec sous pression réduite (20 mm de mercure; 2,7 kPa) à 40° C et le résidu est chromatographié sur une colonne de 7 cm de diamètre contenant 1250 g de silice (0,06–0,2 mm) en éluant par de l'acétate d'éthyle et en recueillant des fractions de 500 $cm^3$. Les 8 premières fractions sont éliminées; les 9 fractions suivantes sont réunies et concentrées à sec sous pression réduite (20 mm de mercure; 2,7 kPa) à 40° C. On obtient ainsi 107 g de produit brut qui sont distillés sous pression réduite (0,7 mm de mercure; 0,09 kPa) à une température comprise entre 135° C et 142° C. Les 33 g de produit obtenus sont chromatographiés sur une colonne de 6 cm de diamètre contenant 480 g de silice (0,040–0,063 mm). On élue par un mélange acétate d'éthyle/cyclohexane (50/50 en volumes) sous une pression de 0,5 bar (51 kPa) ein recueillant des fractions de 250 $cm^3$. Les 7 premières fractions sont éliminées; les 10 fractions suivants sont réunies et concentrées à sec sous pression réduite (1 mm de mercure; 0,13 kPa) à une température de 60° C. On obtient ainsi 27,8 g d'isobutyl-7 oxo-5 tétrahydro-2,3,6,7 5H-thiazolo [3,2-a] pyrimidine sous forme d'huile jaune pâle (indice de réfraction: $n_D^{23}$ = 1,545).

## Exemple 10

Une solution de 342 g de chlorure de sorboyle dans 1600 $cm^3$ de chloroforme est ajoutée goutte à goutte en 75 minutes à une solution de 294 g d'amino-2 thiazoline-2 et de 368 $cm^3$ de triéthylamine dans

1600 cm³ de chloroforme. Le mélange réactionnel, dont la température est alors de 52° C, est chauffé au reflux pendant 16 heures. Après refroidissement à 20° C environ, les cristaux apparus sont séparés par filtration et éliminés. Le filtrat organique est concentré à sec sous pression réduite (20 mm de mercure; 2,7 kPa) à 35° C puis repris par 1200 cm³ d'acétone. Les cristaux apparus à nouveau sont séparés par filtration et éliminés. Le filtrat organique est concentré à sec sous pression réduite (20 mm de mercure; 2,7 kPa) à 40° C et le résidu est chromatographié sur une colonne de 6,5 cm de diamètre contenant 1620 g d'alumine neutre (0,12—0,15 mm) en éluant par du chlorure de méthylène et en recueillant des fractions de 300 cm³. Les 2 premières fractions sont éliminées; les 5 fractions suivantes sont réunies et concentrées à sec sous pression réduite (20 mm de mercure; 2,7 kPa) à 40° C. On obtient ainsi 56 g de produit brut sous forme d'huile que l'on chromatographie à nouveau sur une colonne de 5 cm de diamètre contenant 560 g de silice (0,063—0,2 mm) en éluant par de l'acétate d'éthyle et en recueillant des fractions de 200 cm³. Les 4 premières fractions sont éliminées; les 8 fractions suivantes sont réunies et concentrées à sec sous pression réduite (20 mm de mercure; 2,7 kPa) à 40° C. On obtient ainsi 33,7 g de produit qui sont ajoutés à 5,2 g de produit préparé de la même manière dans une autre opération. Par distillation sous pression réduite (0,8 mm de mercure; 0,11 kPa), on obtient 14,8 g de produit bouillant à 165° C. Après addition de 5 mg d'hydroquinone, le produit est redistillé sous pression réduite (3,5 mm de mercure; 0,47 kPa) sous atmosphère d'azote. On obtient ainsi 9,5 g d'oxo-5 (propène-1yl)-7 tétrahydro-2,3,6,7 5H-thiazolo [3,2-a] pyrimidine sous forme d'huile jaune pâle bouillant à 202° C (indice de réfraction: $n_D^{23}$ =1,581).

## Exemple 11

Une solution de 24 g de chlorure de cyclopropyl-3 acryloyle dans 90 cm³ de chloroforme est ajouté goutte à goutte en 15 minutes à une solution de 21 g d'amino-2 thiazoline-2 et de 25,9 cm³ de triéthylamine dans 90 cm³ de chloroforme. Le mélange réactionnel, dont la température est alors de 63° C, est chauffé au reflux pendant 18 heures. Après refroidissement à 20° C environ, les cristaux apparus sont séparés par filtration et éliminés. Le filtrat organique est concentré à sec sous pression réduite (20 mm de mercure; 2,7 kPa) à 40° C puis repris par 260 cm³ d'acétone. Les cristaux apparus à nouveau sont séparés par filtration, lavés 2 fois par 100 cm³ au total d'acétone et éliminés. Les filtrats organiques réunis sont concentrés à sec sous pression réduite (20 mm de mercure; 2,7 kPa) à 30° C et le résidu est chromatographié sur une colonne de 4,7 cm de diamètre contenant 400 g de silice (0,063—0,2 mm) en éluant par un mélange d'acétate d'éthyle et de cyclohexane (50/50 en volumes) et en recueillant des fractions de 300 cm³. Les 8 premières fractions sont éliminées, les 5 fractions suivantes sont réunies et concentrées à sec sous pression réduite (20 mm de mercure; 2,7 kPa) à 40° C. On obtient ainsi 6 g de produit brut qui sont distillés sous pression réduite (2,5 mm de mercure; 0,34 kPa). On obtient ainsi 4,4 g de produit bouillant à 172° C. Après addition de 15 cm³ d'oxyde d'isopropyle et refroidissement à une température voisine de 5° C, les cristaux apparus sont séparés par filtration, lavés 2 fois par 5 cm³ au total d'oxyde d'isopropyle refroidi à 5° C environ et séchés sous pression réduite (20 mm de mercure; 2,7 kPa) à une température voisine de 20° C en présence de potasse en pastilles. On obtient ainsi 3,5 g de cyclopropyl-7 oxo-5 tétrahydro-2,3,6,7 5H-thiazolo [3,2-a] pyridime sous forme de cristaux blancs fondant à 60° C.

## Exemple 12

Une solution de 83,4 g de chlorure de cyclohexyl-3 acryloyle dans 340 cm³ de chloroforme est ajoutée goutte à goutte en 30 minutes à une solution de 55,4 g d'amino-2 thiazoline-2 et de 68 cm³ de triéthylamine dans 340 cm³ de chloroforme. Le mélange réactionnel, dont la température est alors de 51° C, est chauffé au reflux pendant 15 heures. Après refroidissement à 20° C environ, les cristaux apparus sont séparés par filtration et éliminés. Le filtrat organique est concentré à sec sous pression réduite (20 mm de mercure; 2,7 kPa) à 45° C puis repris par 700 cm³ d'acétone. Les cristaux apparus à nouveau sont séparés par filtration et éliminés. Le filtrat organique est concentré à sec sous pression réduite (20 mm de mercure; 2,7 kPa) à 40° C et le résidu est chromatographié sur une colonne de 6,4 cm de diamètre contenant 1 kg de silice (0,06—0,2 mm) en éluant par de l'acétate d'éthyle et en recueillant des fractions de 1000 cm³. La première fraction est éliminée; les 3 fractions suivantes sont réunies et concentrées à sec sous pression réduite (20 mm de mercure; 2,7 kPa) à 40° C. On obtient ainsi 72 g de produit brut qui sont à nouveau chromatographiés sur une colonne de 4,2 cm de diamètre contenant 610 g d'alumine neutre (0,12—0,15 mm). On élue par un mélange chloroforme/cyclohexane (50/50 en volumes) en recueillant des fractions de 250 cm³. La première fraction est éliminée; les 9 fractions suivantes sont réunies et concentrées à sec sous pression réduite (20 mm de mercure; 2,7 kPa) à 40° C. On obtient ainsi 50,7 g de produit que l'on dissout dans 250 cm³ d'oxyde d'isopropyle bouillant additionnés de 0,5 g de noir décolorant. Après filtration à chaud, le filtrat est refroidi à 4° C pendant 3 heures; les cristaux apparus sont séparés par filtration, lavés 2 fois par 30 cm³ au total d'oxyde d'isopropyle refroidi à 5° C environ et séchés sous pression reduite (20 mm de mercure; 2,7 kPa) à une

température voisine de 20° C en présence de potasse en pastilles. On obtient ainsi 40,7 g de cyclohexyl-7 oxo-5 tétrahydro-2,3,6,7 5H-thiazolo [3,2-a] pyrimidine sous forme de cristaux blancs fondant à 90° C.

Exemple 13

Une solution de 72,2 g de chlorure de méthacryloyle dans 480 $cm^3$ de chloroforme est ajoutée goutte à goutte en 50 minutes à une solution de 82,8 g d'amino-2 thiazoline-2 et de 97 $cm^3$ de triéthylamine dans 400 $cm^3$ de chloroforme. Le mélange réactionnel, dont la température est alors de 54° C, est chauffé au reflux pendant 1 heure. Après refroidissement à 20° C environ, les cristaux apparus sont séparés par filtration et éliminés. Le filtrat organique est concentré à sec sous pression réduite (20 mm de mercure; 2,7 kPa) à 45° C puis repris par 400 $cm^3$ d'acétate d'éthyle. Les cristaux apparus à nouveau sont séparés par filtration, lavés 2 fois par 100 $cm^3$ au total d'acétate d'éthyle et éliminés. L'ensemble des solutions organiques est concentré à sec sous pression réduite (20 mm de mercure; 2,7 kPa) à 40° C et le résidu est chromatographié sur une colonne de 6,2 cm de diamètre contenant 1 kg de silice (0,06 – 0,2 mm) en éluant par des mélanges acétate d'éthyle/cyclohexane et en recueillant des fractions de 500 $cm^3$. Les 8 premières fractions provenant de l'élution par un mélange 20/80 en volumes ainsi que les 7 suivantes provenant de l'élution par un mélange 25/75 en volumes, sont éliminées. Les 8 fractions suivantes provenant de l'élution par un mélange 30/70 en volumes sont réunies et concentrées à sec sous pression réduite (20 mm de mercure; 2,7 kPa) à 40° C. On obtient ainsi 27,9 g de produit qui sont divisés en 2 lots égaux pour être chromatographiés séparément, chacun sur une colonne de 5 cm de diamètre contenant 300 g de silice (0,040 – 0,063 mm). On élue par de l'acétate d'éthyle sous une pression de 0,5 bar (51 kPa) en recueillant des fractions de 75 $cm^3$. Les 7 premières fractions sont éliminées; les 6 fractions suivantes sont réunies et concentrées à sec sous pression réduite (20 mm de mercure; 2,7 kPa) à 40° C. On obtient ainsi 8 g de produit que l'on dissout dans 30 $cm^3$ d'un mélange d'acétate d'éthyle et d'hexane (40/60 en volumes) bouillant additionné de 0,5 g de noir décolorant. Après filtration à chaud, le filtrat est refroidi à 4° C pendant 4 heures; les cristaux apparus sont séparés par filtration, lavés par 10 $cm^3$ du mélange acétate d'éthyle/hexane (40/60 en volumes) et séchés sous pression réduite (20 mm de mercure; 2,7 kPa) à une température voisine de 20° C en présence de potasse en pastilles. On obtient ainsi 3,9 g de méthyl-6 oxo-5 tétrahydro-2,3,6,7 5H-thioazolo [3,2-a] pyrimidine sous forme de cristaux blancs fondant à 76° C.

Exemple 14

Une solution de 48,5 g de chlorure de diméthyl-3,3 aryloyle dans 300 $cm^3$ de chloroforme est ajoutée goutte à goutte en 15 minutes à une solution de 46,6 g d'amino-2 thiazoline-2 et de 54,5 $cm^3$ de triéthylamine dans 300 $cm^3$ de chloroforme. Le mélange réactionnel, dont la température est alors de 51° C, est chauffé au reflux pendant 17 heures. Après refroidissement à 20° C environ, les cristaux apparus sont séparés par filtration et éliminés. Le filtrat organique est concentré à sec sous pression réduite (20 mm de mercure; 2,7 kPa) à 40° C puis repris par 250 $cm^3$ d'acétate d'éthyle. Les cristaux apparus à nouveau sont séparés par filtration, lavés 2 fois par 100 $cm^3$ au total d'acétate d'éthyle et éliminés. Les filtrats organiques sont réunis et concentrés à sec sous pression réduite (20 mm de mercure; 2,7 kPa) et le résidu est chromatographié sur une colonne de 7,5 cm de diamètre contenant 1,25 kg de silice (0,063 – 0,2 mm) en éluant par de l'acétate d'éthyle et en recueillant des fractions de 500 $cm^3$. Les 9 premières fractions sont éliminées; les 8 fractions suivantes sont réunies et concentrées à sec sous pression réduite (20 mm de mercure; 2,7 kPa) à 40° C. On obtient ainsi 37 g de produit sous forme d'un solide marron clair que l'on dissout dans 190 cm3 d'acétate d'éthyle bouillant additionné de 0,3 g de noir décolorant. Après filtration à chaud, le filtrat est refroidi à 4° C pendant 2 heures. Les cristaux apparus sont séparés par filtration, lavés 2 fois par 20 $cm^3$ au total d'acétate d'éthyle refroidi à 5° C environ et éliminés. Les filtrats organiques sont réunis et concentrés à sec sous pression réduite (20 mm de mercure; 2,7 kPa) puis le résidu est dissous dans 140 $cm^3$ d'oxyde d'isopropyle bouillant additionné de 1 g de noir décolerant. Après filtration à chaud, le filtrat est refroidi à 4° C pendant 2 heures; les cristaux apparus sont séparés par filtration, lavés 2 fois par 20 $cm^3$ au total d'oxyde d'isopropyle refroidi à 5° C environ et séchés sous pression réduite (20 mm de mercure; 2,7 kPa) à une température voisine de 20° C en présence de potasse en pastilles. On obtient ainsi 13,8 g de produit brut fondant à 92° C. Après dissolution dans 150 $cm^3$ d'eau, et élimination par filtration de l'insoluble résiduel, le filtrat est lyophilisé. On obtient ainsi 12,2 g de produit que l'on dissout dans 110 $cm^3$ d'oxyde d'isopropyle bouillant. Après refroidissement à 4° C pendant 90 minutes, les cristaux apparus sont séparés par filtration, lavés 2 fois par 20 $cm^3$ au total d'oxyde d'isopropyle refroidi à 5° C environ et séchés sous pression réduite (20 mm de mercure; 2,7 kPa) à une température voisine de 20° C en présence de potasse en pastilles. On obtient ainsi 11,1 g de diméthyl-7,7 oxo-5 tétrahydro-2,3,6,7 5H-thiazolo [3,2-a] pyrimidine sous forme de cristaux crème fondant à 90° C.

Exemple 15

Une solution de 23,6 g de chlorure de crotonoyle dans 100 cm³ de chloroforme est ajoutée goutte à goutte en 20 minutes à une solution de 27,1 g d'amino-2 méthyl-4 thiazoline-2 et de 31,8 cm³ de triéthylamine dans 300 cm³ de chloroforme. Le mélange réactionnel, dont la température est alors de 38° C, est chauffé au reflux pendant 3 heures. Après refroidissement à 40° C environ, le mélange réactionnel est concentré à sec sous pression réduite (20 mm de mercure; 2,7 kPa) à 40° C puis repris par 200 cm³ d'acétone. Les cristaux apparus sont séparés par filtration, lavés 2 fois par 20 cm³ au total d'acétone et éliminés. Les filtrats organiques sont réunis et concentrés à sec sous pression réduite (20 mm de mercure; 2,7 kPa) à 40° C et le résidu est chromatographié sur une colonne de 4,2 cm de diamètre contenant 320 g de silice (0,063 – 0,2 mm). On élue par de l'acétate d'éthyle en recueillant des fractions de 250 cm³. Les 6 premières fractions sont éliminées; les 4 fractions suivantes sont réunies et concentrées à sec sous pression réduite (20 mm de mercure; 2,7 kPa). On obtient ainsi 11,1 g de produit brut sous forme d'huile, qui sont ajoutés à 4 g de produit préparé de la même manière dans une autre opération puis divisés en 3 lots égaux pour être chromatographiés séparément, chacun sur une colonne de 4 cm de diamètre contenant 150 g de silice (0,040 – 0,063 mm) On élue par un mélange d'acétate d'éthyle et de cyclohexane (90/10 en volumes) sous une pression de 0,5 bar (51 kPa) en recueillant des fractions de 50 cm³. Les 9 premières fractions sont éliminées; les 9 fractions suivantes sont réunies et concentrées à sec sous pression réduite (20 mm de mercure; 2,7 kPa) à 40° C. On obtient ainsi 8 g de produit que l'on dissout dans 40 cm³ d'oxyde d'isopropyle bouillant. Après refroidissement à 4° C pendant 1 heure, les cristaux apparus sont sépraés par filtration, lavés par 10 cm³ d'oxyde d'isopropyle refroidi à 5° C environ, et séchés sous pression réduite (20 mm de mercure; 2,7 kPa) à une température voisine de 20° C en présence de potasse en pastilles. On obtient ainsi 6,2 g de diméthyl-3,7 oxo-5 tétrahydro-2,3,6,7 5H-thiazolo [3,2-a] pyrimidine sous forme de cristaux blancs fondant à 76° C.

Exemple 16

Une solution de 89 g de chlorure de (chloro-1 cyclohexane) acétyle dans 350 cm³ de chloroforme est ajoutée goutte à goutte en 35 minutes à une solution de 52,7 g d'amino-2 thiazoline-2 et de 130 cm³ de triéthylamine dans 350 cm³ de chloroforme. Le mélange réactionnel, dont la température est alors de 42° C, est chauffé au reflux pendant 16 heures. Après refroidissement à 20° C environ, les cristaux apparus sont séparés par filtration et éliminés. Le filtrat organique est concentré à sec sous pression réduite (20 mm de mercure; 2,7 kPa) à 40° C puis repris par 400 cm³ d'acétone. Les cristaux apparus à nouveau sont séparés par filtration, lavés 2 fois par 50 cm³ au total d'acétone et éliminés. Les filtrats organiques sont réunis et concentrés à sec sous pression réduite (20 mm de mercure; 2,7 kPa) à 40° C et le résidu est chromatographié sur une colonne de 5 cm de diamètre contenant 880 g d'alumine neutre (0,12 – 0,15 mm) en éluant par un mélange d'acétate d'éthyle et de cyclohexane (50/50 en volumes) et en recueillant des fractions de 250 cm³. Les 2 premières fractions sont éliminées; les 3 fractions suivantes sont réunies et concentrées à sec sous pression réduite (20 mm de mercure; 2,7 kPa) à 40° C. On obtient ainsi 35,6 g de produit brut. Ce produit est chromatographié une seconde fois sur une colonne de 3,7 cm de diamètre contenant 360 g d'alumine neutre (0,12 – 0,15 mm) en éluant par du cyclohexane et en recueillant des fractions de 100 cm³. Après élimination des 6 premières fractions et concentration des 9 fractions suivantes sous pression réduite (20 mm de mercure; 2,7 kPa), on obtient 15 g de produit. Une troisième chromatographie sur une colonne de 6 cm de diamètre contenant 500 g de silice (0,040 – 0,063 mm) est effectuée en éluant par un mélange de cyclohexane et d'acétate d'éthyle (45/55 en volumes) sous une pression de 0,5 bar (51 kPa) et en recueillant des fractions de 100 cm³. Après élimination des 12 premières fractions et concentration sous suivantes, on isole 5,4 g d'un solide jaune que l'on dissout alors dans un mélange de 60 cm³ d'oxyde d'isopropyle et de 2 cm³ d'acétonitrile bouillant additionné de 0,05 g de noir décolorant. Après filtration à chaud, le filtrat est refroidi à 4° C pendant 16 heures, les cristaux apparus sont séparés par filtration, lavés 2 fois par 20 cm³ au total d'oxyde d'isopropyle refroidi à 5° C environ, et séchés sous pression réduite (20 mm de mercure; 2,7 kPa) à une température voisine de 20° C en présence de potasse en pastilles. On obtient finalement 4,4 g d'oxo-5 tétrahydro-2,3,6,7 spiro [[5H-thiazolo [3,2-a] pyrimidine-7,1'-cyclohexane]] sous forme de cristaux blancs, fondant à 110° C.

Le chlorure de (chloro-1 cyclohexane) acétyle peut être préparé de la façon suivante:

14 g d'acide cyclohexanol-1 acétique en solution dans 100 cm³ de chloroforme et 1 cm³ de diméthylformamide sont additionnés goutte à goutte de 14,5 cm³ de chlorure de thionyle en 15 minutes, puis chauffés progressivement au reflux jusqu'à la fin du dégagement gazeux. Le mélange réactionnel est alors concentré par distillation du chloroforme et de l'excès de chlorure de thionyle sous pression atmosphérique (760 mm de mercure environ; 101 kPa). Après reprise à l'aide de 50 cm³ de cyclohexane et nouvelle concentration sous pression atmosphérique, on obtient 17,1 g de chlorure de (chloro-1 cyclohexane) acétyle sous forme d'une huile marron.

Exemple 17

Une solution de 48 g de chlorure de chloro-3 trifluoro-4,4,4 butyryle dans 150 $cm^3$ de chloroforme est ajoutée goutte à goutte en 30 minutes à une solution de 27,8 g d'amino-2 thiazoline-2 et de 70 $cm^3$ de triéthylamine dans 400 $cm^3$ de chloroforme. Le mélange réactionnel, dont la température est alors de 60° C, est chauffé au reflux pendant 1 heure. Après refoidissement à 20° C environ, les cristaux apparus sont séparés par filtration, lavés 2 fois par 40 $cm^3$ au total de chloroforme et éliminés. Les filtrats organiques sont réunis et repris par 400 $cm^3$ d'acétone. Les cristaux apparus à nouveau sont séparés par filtration, lavés 2 fois par 100 $cm^3$ au total d'acétone et éliminés. Les filtrats organiques sont réunis et concentrés à sec sous pression réduite (20 mm de mercure; 2,7 kPa) à 35°C et le résidu est chromatographié sur une colonne de 5 cm de diamètre contenant 700 g de silice (0,063–0,2 mm) en éluant par un mélange d'acétate d'éthyle et de cyclohexane (40/60 en volumes) et en recueillant des fractions de 500 $cm^3$. La première fraction est éliminée, les 4 fractions suivantes sont réunies et concentrées à sec sous pression réduite (20 mm de mercure; 2,7 kPa) à 40°C. On obtient ainsi 8 g de produit brut qui sont chromatographiés sur une colonne de 4 cm de diamètre contenant 150 g de silice (0,040–0,063 mm). On élue par un mélange d'acétate d'éthyle et de cyclohexane (45/55 en volumes) sous une pression de 0,5 bar (51 kPa) en recueillant des fractions de 10 $cm^3$. Les 16 premières fractions sont éliminées, les 8 fractions suivantes sont réunies et concentrées à sec sous pression réduite (20 mm de mercure; 2,7 kPa) à 40° C. On obtient ainsi 2 g de produit. Après addition de 0,6 g de produit préparé de la même manière dans une autre opération, dissolution dans 30 $cm^3$ d'oxyde d'isopropyle bouillant additionné de 0,05 g de noir décolorant et filtration à chaud, on refroidit le filtrat à 5°C pendant 16 heures. Les cristaux apparus sont séparés par filtration, lavés 2 fois par 10 $cm^3$ au total d'oxide d'isopropyle refroidi à 5°C environ et séchés sous pression réduite (20 mm de mercure; 2,7 kPa) à une température voisine de 20° C en présence de potasse en pastilles. On obtient ainsi 1,7 g d'oxo-5 trifluorométhyl-7 tétrahydro-2,3,6,7 5H-thiazolo [3,2-a] pyrimidine sous forme de cristaux blancs fondant à 120° C.

La présente invention concerne egalement les médicaments constitués par au moins un produit de formule générale (I), à l'état pur ou sous forme d'une composition dans laquelle il est associé à tout autre produit pharmaceutiquement compatible, pouvant être inerte ou physiologiquement actif. Les médicaments selon l'invention peuvent être utilisés par voie orale, parentérale ou rectale.

Comme compositions solides pour administration orale peuvent être utilisés des comprimés, pilules, poudres (notamment dans des capsules de gélatine ou des cachets) ou granulés. Dans ces compositions, le produit actif selon l'invention est mélangé à un ou plusieurs diluants inertes, tels que amidon, cellulose, saccharose, lactose ou silice. Ces compositions peuvent également comprendre des substances autres que les diluants, par exemple un ou plusieurs lubrifiants tel que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent également comprendre des substances autres que les diluants, par exemple des produits mouillants, édulcorants, épaississants, aromatisants ou stabilisants.

Les compositions stériles pour administration parentérale peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou autres solvants organiques convenables. Ces compositions peuvent également vontenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chaffage. Elles peuvent également être préparées sous forme de compositions solides stériles qui peuvent être dissoutes au moment de l'emploi dans un milieu stérile injectable.

Les compositions pour administration rectale sont les suppositoires ou les capsules rectales, qui contiennent outre le produit actif des excipients tels que le beurre de cacao, des glycérides semi-synthétiques ou des polyéthylèneglycols.

En thérapeutique humaine, les produits de l'invention sont particulièrement utiles dans le traitement de fond de la maladie rhumatismale et des états allergisques. Les doses dépendent de l'effet recherché et de la durée du traitement; elles sont généralement comprises entre 50 et 1000 mg par jour par voie orale pour un adulte en une ou plusieurs prises.

D'une façon générale, le médicin déterminera la posologie qu'il estime la plus appropriée en fonction de l'âge, du poids et de tous les austres facteurs propres au sujet à traiter.

Les exemples suivants, donnés à titre non limitatif, illustrent une composition selon l'invention.

Exemple A

On prépare selon la technique habituelle des comprimés dosés à 50 mg de produit actif ayant la composition suivante:

| | |
|---|---|
| – oxo-5-tétrahydro-2,3,6,7-spiro[[5H-thiazolo[3,2-a]pyrimidine-7,1′-cyclohexane]] | 50 mg |
| – amidon | 60 mg |
| – lactose | 50 mg |
| – stéarate de magnésium | 2 mg |

Exemple B

On prépare selon la technique habituelle des comprimés dosés à 50 mg de produit actif ayant la composition suivante:

| | |
|---|---|
| – Méthyl-7-oxo-5-tétrahydro-2,3,6,7-5H-thiazolo[3,2-a]pyrimidine | 50 mg |
| – amidon | 15 mg |
| – silice colloïdale | 9,5 mg |
| – stéarate de magnésium | 0,5 mg |

**Revendications pour les etats contractants: BE, CH, DE, FR, GB, IT, LI, LU, NL, SE**

1. Nouveau dérivé de la tétrahydro-2,3,6,7 thiazolo [3,2-a] pyrimidinone-5, caractérisé en ce qu'il répond à la formule générale:

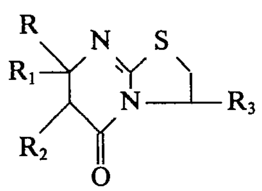

dans laquelle
– soit R représente un radical phényle, alcoyle contenant 1 à 4 atomes de carbone non substitué ou substitué par 1 à 3 atomes d'halogène, alcényle contenant 2 à 4 atomes de carbone ou cycloalcoyle contenant 3 à 6 atomes de carbone et $R_1$ représente un atome d'hydrogène, ou bien R et $R_1$, identiques ou différents, représentent chacun un radical phényle ou alcoyle contenant 1 à 4 atomes de carbone non substitué, ou forment ensemble un radical alcoylène contenant 4 ou 5 atomes de carbone et $R_2$ et $R_3$, identiques ou différents, représentent un atome d'hydrogène ou un radical alcoyle contenant 1 à 4 atomes de carbone,
– soit R et $R_1$ représentent chacun un atome d'hydrogène et $R_2$ et $R_3$ représentent l'un un atome d'hydrogène et l'autre un radical alcoyle contenant 1 à 4 atomes de carbone ou bien représentent chacun un tel radical alcoyle.

2. Procédé de préparation d'un produit selon la revendication 1, caractérisé en ce que l'on fait agir un halogénure de formule générale:

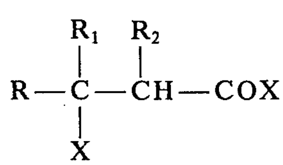

sur une amino-2 thiazoline-2 (en équilibre avec sa forme imine) de formule générale:

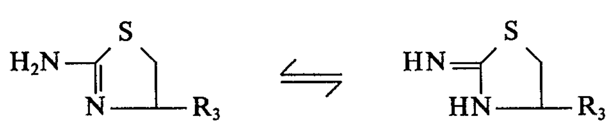

dans lesquelles R, $R_1$, $R_2$ et $R_3$ sont définis comme dans la revendication 1 et X représente un atome d'halogène, puis on isole le produit obtenu.

3. Procédé de préparation d'un produit selon la revendication 1, à l'exception de ceux pour lesquels R et $R_1$ forment ensemble un radical alcoylène, caractérisé en ce que l'on fait agir un halogénure de formule générale:

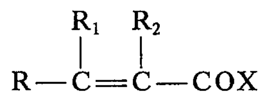

sur une amino-2 thiazoline-2 (en équilibre avec sa forme imine) de formule générale:

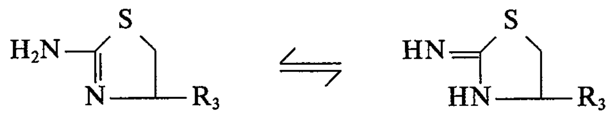

dans lesquelles R, $R_1$, $R_2$ et $R_3$ sont définis comme dans la revendication 1 et X représente un atome d'halogène, puis on isole le produit obtenu.

4. Procédé préparation d'un produit selon la revendication 1, à l'exception de ceux pour lesquels R et $R_1$ forment ensemble un radical alcoylène, caractérisé en ce que l'on fait agir un ester de formule générale:

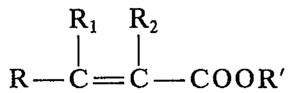

sur une amino-2 thiazoline-2 (en équilibre avec sa forme imine) de formule générale:

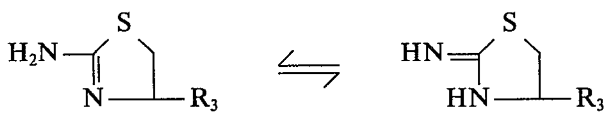

dans lesquelles R, $R_1$, $R_2$ et $R_3$ sont définis comme dans la revendication 1 et R' représente un radical alcoyle contenant 1 à 4 atomes de carbone, puis on isole le produit obtenu.

5. Procédé de préparation d'un produit selon la revendication 1 dans laquelle $R_3$ représente un atome d'hydrogène, caractérisé en ce que l'on fait réagir le dibromo-1,2 éthane sur un dihydro-4,5 thio-2 uracile de formule générale:

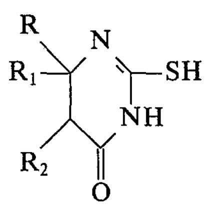

dans laquelle R, $R_1$ et $R_2$ sont définis comme dans la revendication 1, puis on isole le produit obtenu.

6. Medicament caractérisé en ce qu'il contient un produit selon la revendication 1 en association avec un ou plusieurs diluants ou adjuvants compatibles et pharmaceutiquement acceptables.

**Revendication pour l'etat contractant: AT**

Procédé de préparation d'un nouveau dérivé de la tétrahydro-2,3,6,7 thiazolo [3,2-a] pyrimidinone-5 de formule générale:

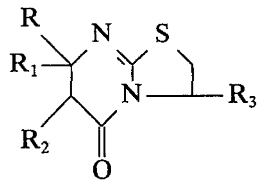

dans laquelle:
– soit R représente un radical phényle, alcoyle contenant 1 à 4 atomes de carbone non substitué ou substitué par 1 à 3 atomes d'halogène, alcényle contenant 2 à 4 atomes de carbone ou cycloalcoyle contenant 3 à 6 atomes de carbone et $R_1$ représente un atome d'hydrogène, ou bien R et $R_1$, identiques ou différents, représentent chacun un radical phényle ou alcoyle contenant 1 à 4 atomes de carbone non substitué, ou forment ensemble un radical alcoylène contenant 4 ou 5 atomes de carbone et $R_2$ et

$R_3$, identiques ou différents, représentent un atome d'hydrogène ou un radical alcoyle contenant 1 à 4 atomes de carbone,

– soit R et $R_1$ représentent chacun un atome d'hydrogène et $R_2$ et $R_3$ représentent l'un un atome d'hydrogène et l'autre un radical alcoyle contenant 1 à 4 atomes de carbone ou bien représentent chacun un tel radical alcoyle, caractérisé en ce que

a) on fait réagir un halogénure de formule générale:

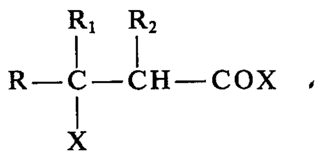

sur une amino-2 thiazoline-2 (en équilibre avec sa forme imine) de formule générale:

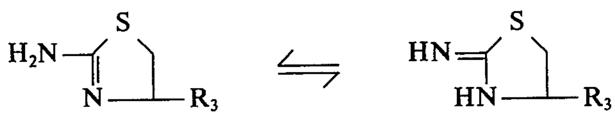

dans lesquelles R, $R_1$, $R_2$ et $R_3$ sont définis comme précédemment et X représente un atome d'halogène, puis on isole le produit obtenu, ou

b) – pour la préparation d'un produit défini comme précédemment, à l'exception de ceux pour lesquels R et $R_1$ forment ensemble un radical alcoylène, on fait agir un halogénure de formule générale:

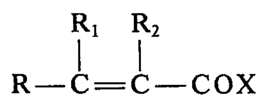

sur une amino-2 thiazoline-2 (en équilibre avec sa forme imine) de formule générale:

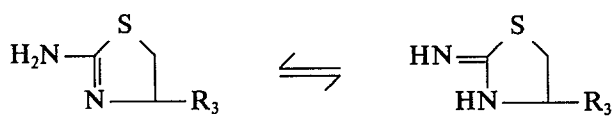

dans lesquelles R, $R_1$, $R_2$ et $R_3$ ont les définitions correspondantes et X représente un atome d'halogène, puis on isole le produit obtenu, ou

c) – pour la préparation d'un produit défini comme précédemment, à l'exception de ceux pour lesquels R et $R_1$ forment ensemble un radical alcoylène, on fait agir un ester de formule générale:

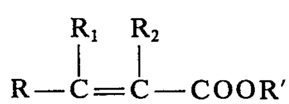

sur une amino-2 thiazoline-2 (en équilibre avec sa forme imine) de formule générale:

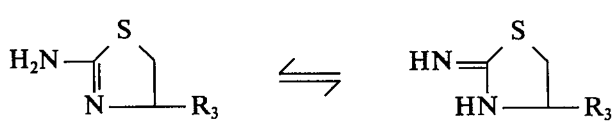

dans lesquelles R, $R_1$, $R_2$ et $R_3$ ont définitions correspondantes et R' représente un radical alcoyle contenant 1 à 4 atomes de carbone, puis on isole le produit obtenu, ou

d) – pour la préparation d'un produit défini comme précédemment dans la formule duquel $R_3$ représente un atome d'hydrogène, on fait réagir le dibromo-1,2 éthane sur un dihydro-4,5 thio-2 uracile de formule générale:

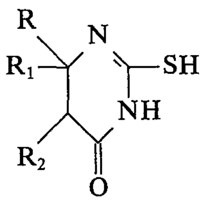

dans laquelle R, $R_1$ et $R_2$ sont définis comme précédemment, puis on isole le produit obtenu.

**Patentansprüche für die Vertragsstaaten: BE, CH, LI, DE, FR, GB, IT, LU, NL, SE**

1. Neues Derivat des 2,3,6,7-Tetrahydro-thiazolo[3,2-a]pyrimidinon-(5), dadurch gekennzeichnet, daß es der allgemeinen Formel

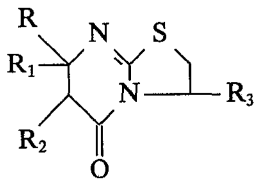

entspricht, worin
– entweder R einen Phenylrest, Alkylrest mit 1 bis 4 Kohlenstoffatomen, nicht substituiert oder substituiert durch 1 bis 3 Halogenatome, Alkenylrest mit 2 bis 4 Kohlenstoffatomen oder Cycloalkylrest mit 3 bis 6 Kohlenstoffatomen bedeutet und $R_1$ ein Wasserstoffatom bedeutet, oder R und $R_1$, die identisch oder voneinander verschieden sind, bedeuten jeweils einen Phenylrest oder nicht substituierten Alkylrest mit 1 bis 4 Kohlenstoffatomen oder bilden zusammen einen Alkylenrest mit 4 oder 5 Kohlenstoffatomen und $R_2$ und $R_3$, welche identisch oder voneinander verschieden sind, bedeuten ein Wasserstoffatom oder einen Alkylrest mit 1 bis 4 Kohlenstoffatomen,
– oder R und $R_1$ bedeuten jeweils ein Wasserstoffatom, und $R_2$ und $R_3$ bedeuten das eine ein Wasserstoffatom und das andere einen Alkylrest mit 1 bis 4 Kohlenstoffatomen oder bedeuten jeweils einen solchen Alkylrest.

2. Verfahren zur Herstellung eines Produkts gemäß Anspruch 1, dadurch gekennzeichnet, daß man ein Halogenid der allgemeinen Formel

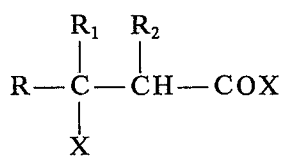

auf ein 2-Aminothiazolin-(2) (im Gleichgewicht mit seiner Iminform) der allgemeinen Formel

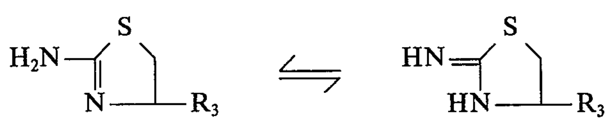

worin R, $R_1$, $R_2$ und $R_3$ wie in Anspruch 1 definiert sind und X ein Halogenatom bedeutet, einwirken läßt und das erhaltene Produkt dann isoliert.

3. Verfahren zur Herstellung eines Produkts gemäß Anspruch 1 mit Ausnahme solcher, für die R und $R_1$ zusammen einen Alkylenrest bilden, dadurch gekennzeichnet, daß man ein Halogenid der allgemeinen Formel

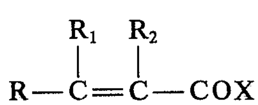

auf ein 2-Aminothiazolin-(2) (im Gleichgewicht mit seiner Iminform) der allgemeinen Formel

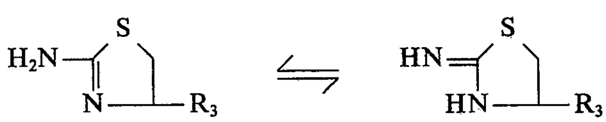

worin R, $R_1$, $R_2$ und $R_3$ wie in Anspruch 1 definiert sind und X ein Halogenatom bedeutet, einwirken läßt und das erhaltene Produkt dann isoliert.

4. Verfahren zur Herstellung eines Produkts gemäß Anspruch 1 mit Ausnahme solcher, für die R und $R_1$ zusammen einen Alkylenrest bilden, dadurch gekennzeichnet, daß man einen Ester der allgemeinen Formel

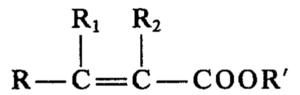

auf ein 2-Aminothiazolin-(2) (im Gleichgewicht mit seiner Iminform der allgemeinen Formel

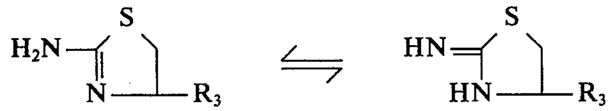

worin R, $R_1$, $R_2$ und $R_3$ wie in Anspruch 1 definiert sind und R' einen Alkylrest mit 1 bis 4 Kohlenstoffatomen bedeutet, einwirken läßt und dann das erhaltene Produkt isoliert.

5. Verfahren zur Herstellung eines Produkts gemäß Anspruch 1, worin $R_3$ ein Wasserstoffatom bedeutet, dadurch gekennzeichnet, daß man 1,2-Dibromäthan auf ein 4,5-Dihydro-2-thiouracil der allgemeinen Formel

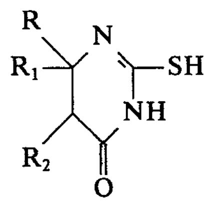

worin R, $R_1$ und $R_2$ wie in Anspruch 1 definiert sind, einwirken läßt und dann das erhaltene Produkt isoliert.

6. Arzneimittel, dadurch gekennzeichnet, daß es ein Produkt gemäß Anspruch 1 zusammen mit einem oder mehreren verträglichen und pharmazeutisch annehmbaren Verdünnungs- oder Hilfsmitteln enthält.

**Patentanspruch für den Vertragsstaat: AT**

Verfahren zur Herstellung eines neuen Derivats des 2,3,6,7-Tetrahydro-thiazolo[3,2-a]pyrimidinons-(5) der allgemeinen Formel

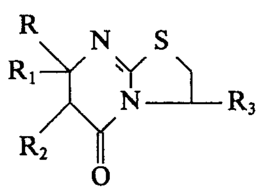

worin:

– entweder R einen Phenylrest, Alkylrest mit 1 bis 4 Kohlenstoffatomen, nicht substituiert oder substituiert durch 1 bis 3 Halogenatome, Alkenylrest mit 2 bis 4 Kohlenstoffatomen oder Cycloalkylrest mit 3 bis 6 Kohlenstoffatomen bedeutet und $R_1$ ein Wasserstoffatom bedeutet oder R und $R_1$, die identisch oder voneinander verschieden sind, bedeuten jeweils einen Phenylrest oder nicht substituierten Alkylrest mit 1 bis 4 Kohlenstoffatomen oder bilden zusammen einen Alkylenrest mit 4 oder 5 Kohlenstoffatomen und $R_2$ und $R_3$, die identisch oder voneinander verschieden sind, bedeuten ein Wasserstoffatom oder einen Alkylrest mit 1 bis 4 Kohlenstoffatomen,

– oder R und $R_1$ bedeuten jeweils ein Wasserstoffatom und $R_2$ und $R_3$ bedeuten das eine ein Wasserstoffatom und das andere einen Alkylrest mit 1 bis 4 Kohlenstoffatomen oder jedes bedeutet einen solchen Alkylrest, dadurch gekennzeichnet, daß man

a) ein Halogenid der allgemeinen Formel

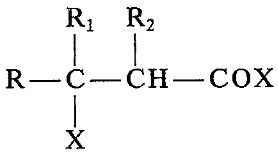

auf ein 2-Amino-thiazolin-(2) (im Gleichgewicht mit seiner Iminform) der allgemeinen Formel

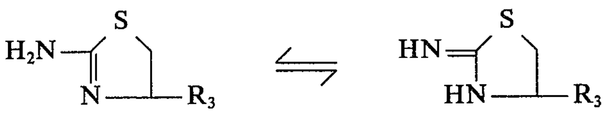

worin R, $R_1$, $R_2$ und $R_3$ wie vorstehend definiert sind und X ein Halogenatom bedeutet, einwirken läßt und dann das erhaltene Produkt isoliert oder

b) – zur Herstellung eines wie vorstehend definierten Produkts mit Ausnahme solcher, für die R und $R_1$ zusammen einen Alkylenrest bilden, ein Halogenid der allgemeinen Formel

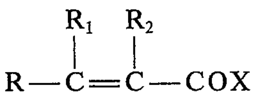

auf ein 2-Amino-thiazolin-(2) (im Gleichgewicht mit seiner Iminform) der allgemeinen Formel

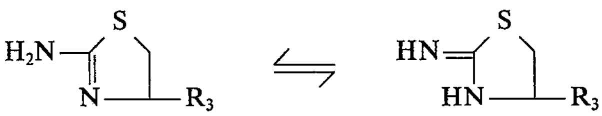

worin R, $R_1$, $R_2$ und $R_3$ die entsprechenden Definitionen haben und X ein Halogenatom bedeutet, einwirken läßt und dann das erhaltene Produkt isoliert oder

c) – zur Herstellung eines wie vorstehend definierten Produkts mit Ausnahme solcher, für die R und $R_1$ zusammen einen Alkylenrest bilden, einen Ester der allgemeinen Formel

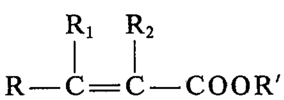

auf ein 2-Amino-thiazolin-(2) (im Gleichgewicht mit seiner Iminform) der allgemeinen Formel

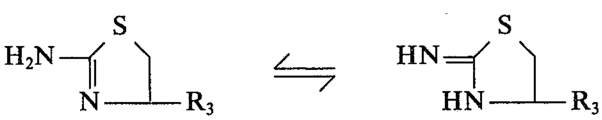

worin R, $R_1$, $R_2$ und $R_3$ die entsprechenden Definitionen haben und R' einen Alkylrest mit 1 bis 4 Kohlenstoffatomen bedeutet, einwirken läßt und dann das erhaltene Produkt isoliert oder

d) – zur Herstellung eines wie vorstehend definierten Produkts, in dessen Formel $R_3$ ein Wasserstoffatom bedeutet, das 1,2-Dibromäthan auf ein 4,5-Dihydro-2-thio-uracil der allgemeinen Formel

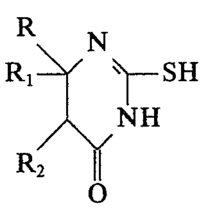

worin R, $R_1$ und $R_2$ wie vorstehend definiert sind, einwirken läßt und das erhaltene Produkt dann isoliert.

**Claims for the contracting states: BE, CH, DE, FR, GB, IT, LI, LU, NL, SE**

1. New 2,3,6,7-tetrahydrothiazolo[3,2-a]pyrimidin-5-one derivative, characterised in that it corresponds to the general formula:

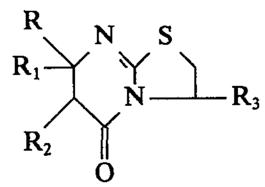

in which
– either R represents a phenyl radical, an alkyl radical containing 1 to 4 carbon atoms, which is unsubstituted or substituted by 1 to 3 halogen atoms, an alkenyl radical containing 2 to 4 carbon atoms or a cycloalkyl radical containing 3 to 6 carbon atoms, and $R_1$ represents a hydrogen atoms, or alternatively R and $R_1$, which are identical or different, each represent a phenyl radical or an unsubstituted alkyl radical containing 1 to 4 carbon atoms, or together form an alkylene radical containing 4 or 5 carbon atoms, and $R_2$ and $R_3$, which are identical or different, represent a hydrogen atom or an alkyl radical containing 1 to 4 carbon atoms,
– or R and $R_1$ each represent a hydrogen atom and $R_2$ and $R_3$ represent in one case a hydrogen atom and in the other an alkyl radical containing 1 to 4 carbon atoms, or alternatively each represent an alkyl radical of this type.

2. Process for the preparation of a product according to Claim 1, characterised in that a halide of the general formula:

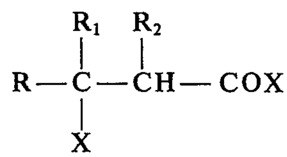

is reacted with a 2-amino-2-thiazoline (in equilibrium with its imine form) of the general formula:

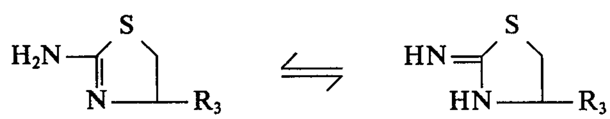

in which formulae R, $R_1$, $R_2$ and $R_3$ are defined as in Claim 1 and X represents a halogen atom, and the product obtained is then isolated.

3. Process for the preparation of a produkt according to Claim 1, with the exception of those in which R and $R_1$ together form an alkylene radical, characterised in that a halide of the general formula:

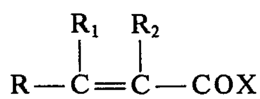

is reacted with a 2-amino-2-thiazoline (in equilibrium with its imine form) of the general formula:

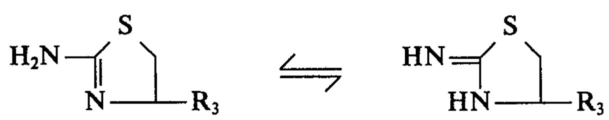

in which formulae R, $R_1$, $R_2$ and $R_3$ are defined as in Claim 1 and X represents a halogen atom, and the product obtained is then isolated.

4. Process for the preparation of a product according to Claim 1, with the exception of those in which R and $R_1$ together form an alkylene radical, characterised in that an ester of the general formula:

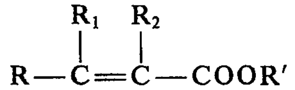

is reacted with a 2-amino-2-thiazoline (in equilibrium with its imine form) of the general formula:

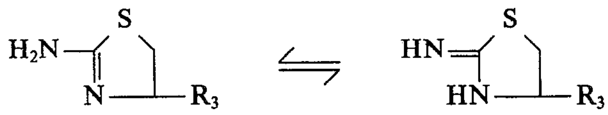

in which formulae R, $R_1$, $R_2$ and $R_3$ are defined as in Claim 1 and R′ represents an alkyl radical containing 1 to 4 carbon atoms, and the product obtained is then isolated.

5. Process for the preparation of a product according to Claim 1 in which $R_3$ represents a hydrogen atom, characterised in that 1,2-dibromoethane is reacted with a 4,5-dihydro-2-thiouracil of the general formula:

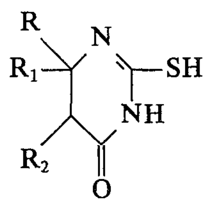

in which R, $R_1$ and $R_2$ are defined as in Claim 1, and the product obtained is then isolated.

6. Medicament, characterised in that it contains a product according to Claim 1 in association with one or more compatible and pharmaceutically acceptable diluents or adjuvants.

**Claim for the contracting state: AT**

Process for the preparation of a new 2,3,6,7-tetrahydrothiazolo[3,2-a]pyrimidin-5-one derivate of the general formula:

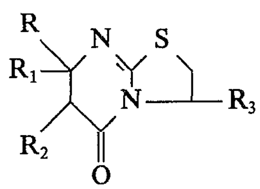

in which:

– either R represents a phenyl radical, an alkyl radical containing 1 to 4 carbon atoms, which is unsubstituted or substituted by 1 to 3 halogen atoms, an alkenyl radical containing 2 to 4 carbon atoms or a cycloalkyl radical containing 3 to 6 carbon atoms, and $R_1$ represents a hydrogen atom, or alternatively R and $R_1$, which are identical or different, each represent a phenyl radical or an unsubstituted alkyl radical containing 1 to 4 carbon atoms, or together form an alkylene radical containing 4 or 5 carbon atoms, and $R_2$ and $R_3$, which are identical or different, represent a hydrogen atom or an alkyl radical containing 1 to 4 carbon atoms,

– or R and $R_1$ each represent a hydrogen atom and $R_2$ and $R_3$ represent in one case a hydrogen atom and in the other an alkyl radical containing 1 to 4 carbon atoms, or alternatively each represent an alkyl radical of this type, characterised in that

a) a halide of the general formula:

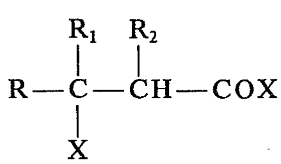

is reacted with a 2-amino-2-thiazoline (in equilibrium with its imine form) of the general formula:

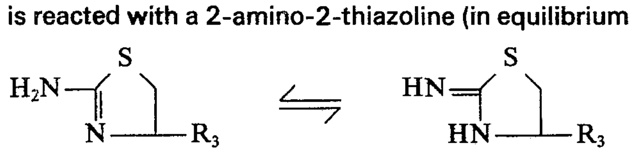

in which formulae R, $R_1$, $R_2$ and $R_3$ are defined as above and X represents a halogen atom, and the product obtained is then isolated, or

b) for the preparation of a produkt defined as above with the exception of those in which R and $R_1$ together form an alkylene radical, a halide of the general formula:

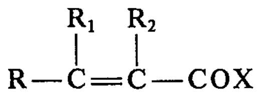

is reacted with a 2-amino-2-thiazoline (in equilibrium with its imine form) of the general formula:

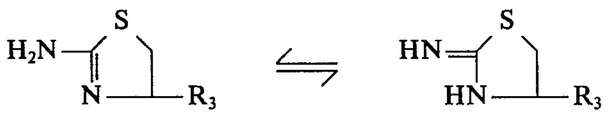

in which formulae R, $R_1$, $R_2$ and $R_3$ have the corresponding definitions and X represents a halogen atom, and the product obtained is then isolated, or

c) for the preparation of a product defined as above, with the exception of those in which R and $R_1$ together form an alkylene radical, an ester of the general formula:

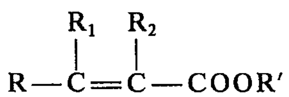

is reacted with a 2-amino-2-thiazoline (in equilibrium with its imine form) of the general formula:

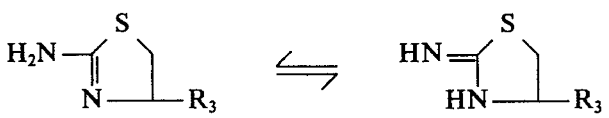

in which formulae R, $R_1$, $R_2$ and $R_3$ have the corresponding definitions and R' represents an alkyl radical containing 1 to 4 carbon atoms, and the produkt obtained is then isolated, or

d) for the preparation of a product defined as above in the formula of which $R_3$ represents a hydrogen atom, 1,2-dibromoethane is reacted with a 4,5-dihydro-2-thiouracil of the general formula:

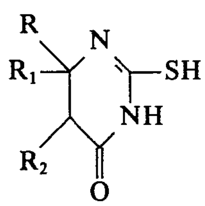

in which R, $R_1$ and $R_2$ are defined as above, and the product obtained is then isolated.